# EUROPÄISCHE PATENTSCHRIFT

(11) **EP 1 519 927 B1**
(45) Veröffentlichungstag und Bekanntmachung des Hinweises auf die Patenterteilung: **05.04.2006**
(21) Anmeldenummer: 03732579.2
(22) Anmeldetag: 17.06.2003
(51) Int. Cl.: C07D 275/03, A01N 43/00

(54) **VERFAHREN ZUR HERSTELLUNG VON 3,4-DICHLOR-N-(2-CYANO-PHENYL)-5-ISOTHIAZOL-CARBOXAMID**
METHOD FOR PRODUCING 3,4-DICHLORO-N-(2-CYANO-PHENYL)-5-ISOTHIAZOLE CARBOXAMIDE
PROCEDE DE PRODUCTION DE 3,4-DICHLORO-N-(2-CYANOPHENYL)-5-ISOTHIAZOLE CARBOXAMIDE

(30) Priorität: 27.06.2002 DE 10228731
(43) Veröffentlichungstag der Anmeldung: 06.04.2005
(73) Patentinhaber: Bayer CropScience AG, 40789 Monheim (DE)
(72) Erfinder: HIMMLER, Thomas, 51519 Odenthal (DE)
(86) Internationale Anmeldenummer: PCT/EP2003/006361
(87) Internationale Veröffentlichungsnummer: WO 2004/002969

(56) Entgegenhaltungen:
- US-B1- 6 277 791
- WANG ET AL.: "TOTAL SYNTHESIS OF THE FUMIQUINAZOLINE ALKALOIDS: SOLUTION-PHASE STUDIES" J. ORG. CHEM., Bd. 65, 2000, Seiten 1022-1030, XP002251326

## Beschreibung

Die vorliegende Erfindung betrifft ein neues Verfahren zur Herstellung des bekannten 3,4-Dichlor-N-(2-cyano-phenyl)-5-isothiazolcarboxamids, das als Wirkstoff mit mikrobiziden Eigenschaften verwendbar ist.

Es ist bereits bekannt geworden, dass man 3,4-Dichlor-N-(2-cyano-phenyl)-5-isothiazolcarboxamid erhält, wenn man 3,4-Dichlor-isothiazol-5-carbonsäurechlorid mit 2-Cyano-anilin umsetzt (vgl. WO 99-24 413). Die Ausbeute bei dieser Herstellungsmethode ist hoch. Nachteilig ist aber, dass das als Ausgangsprodukt benötigte 2-Cyano-anilin nur durch eine aufwendige Synthese zugänglich ist (vgl. DE-A 2 115 624 und DE-A 2 115 625). So ist es erforderlich, zunächst Anthranilsäureamid in Gegenwart von Dimethylformamid mit Phosgen umzusetzen und das dabei entstehende N-2-Cyano-phenyl-N',N'-dimethyl-formamidinium-Hydrochlorid dann in einem zweiten Schritt mit Natriumacetat in wässrigem Medium zu behandeln.

Es wurde nun gefunden, dass man 3,4-Dichlor-N-(2-cyano-phenyl)-5-isothiazolcarboxamid der Formel 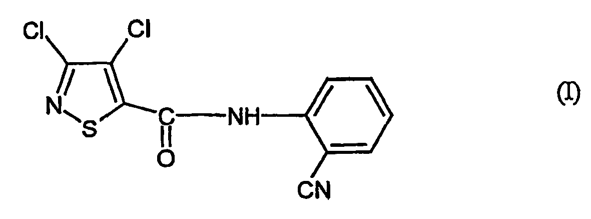 nach einem Eintopf-Verfahren erhält, indem man
a) zunächst Isatosäureanhydrid der Formel 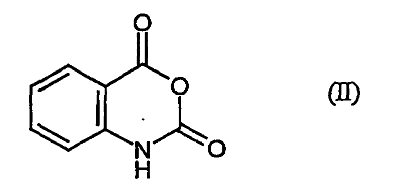 in Gegenwart von Dimethylformamid mit Ammoniakgas umsetzt,
b) dann das dabei entstandene Anthranilsäureamid der Formel 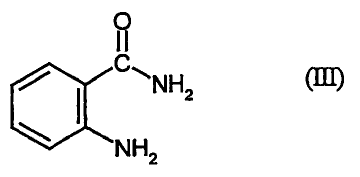 ohne vorherige Isolierung mit 3,4-Dichlor-isothiazolcarbonsäurechlorid der Formel 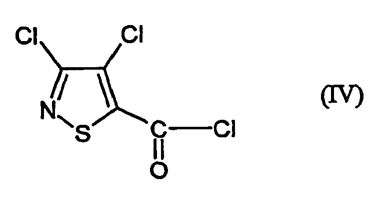 in Gegenwart eines Säureakzeptors und in Gegenwart von Dimethylformamid umsetzt und
c) anschließend das dabei entstandene N-[2-(Aminocarbonyl)-phenyl]-3,4-dichlor-isothiazolcarboxamid der Formel 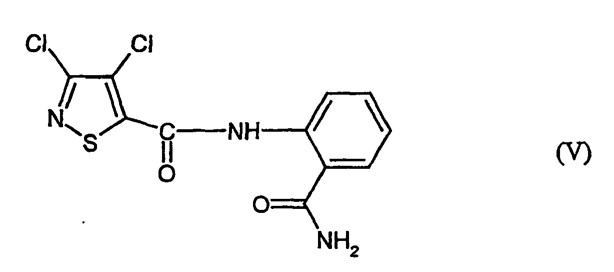 ohne vorherige Isolierung in Gegenwart von Dimethylformamid mit Thionylchlorid, Phosphoroxychlorid, Phosgen oder Chlormethylen-dimethylammoniumchlorid umsetzt.

Es ist als ausgesprochen überraschend zu bezeichnen, dass sich 3,4-Dichlor-N-(2-Cyano-phenyl)-5-isothiazolcarboxamid der Formel (I) nach dem erfindungsgemäßen Verfahren in glatter Reaktion ohne störende Nebenreaktionen herstellen lässt. So musste aufgrund des vorbekannten Standes der Technik damit gerechnet werden, dass N-acylierte Anthranilsäurederivate unter sauren, basischen oder neutralen Bedingungen unter Abspaltung von Wasser leicht zu Benzoxazinonen beziehungsweise Chinazolinolen oder Chinazolinonen cyclisiert werden (vgl. Farmaco Ed. Sci. 39 (1984), 120; J. Heterocycl. Chem. 16 (1979) 711; J. prakt Chem. 111 (1925) 48 und Egypt. J. Chem 31 (1988) 241). Im Gegensatz zu den Erwartungen wird die erfindungsgemäße Umsetzung aber nicht durch derartige unerwünschte Kondensationen beeinträchtigt.

Das erfindungsgemäße Verfahren zeichnet sich durch eine Reihe von Vorteilen aus. So ermöglicht es die Synthese des 3,4-Dichlor-N-(2-cyanophenyl)-5-isothiazolcarboxamids der Formel (I) in hoher Ausbeute und hervorragender Reinheit. Günstig ist auch, dass die Durchführung der Umsetzung und die Isolierung des Reaktionsproduktes keinerlei Schwierigkeiten bereitet. Außerdem lässt sich das erfindungsgemäße Verfahren problemlos in den technischen Maßstab übertragen.

Verwendet man bei der Durchführung des erfindungsgemäßen Verfahrens in der zweiten Stufe Triethylamin als Säureakzeptor und in der dritten Stufe Thionylchlorid im Gemisch mit Dimethylformamid als Dehydratisierungsmittel, so kann der Verlauf der Umsetzungen durch das folgende Formelschema veranschaulicht werden.

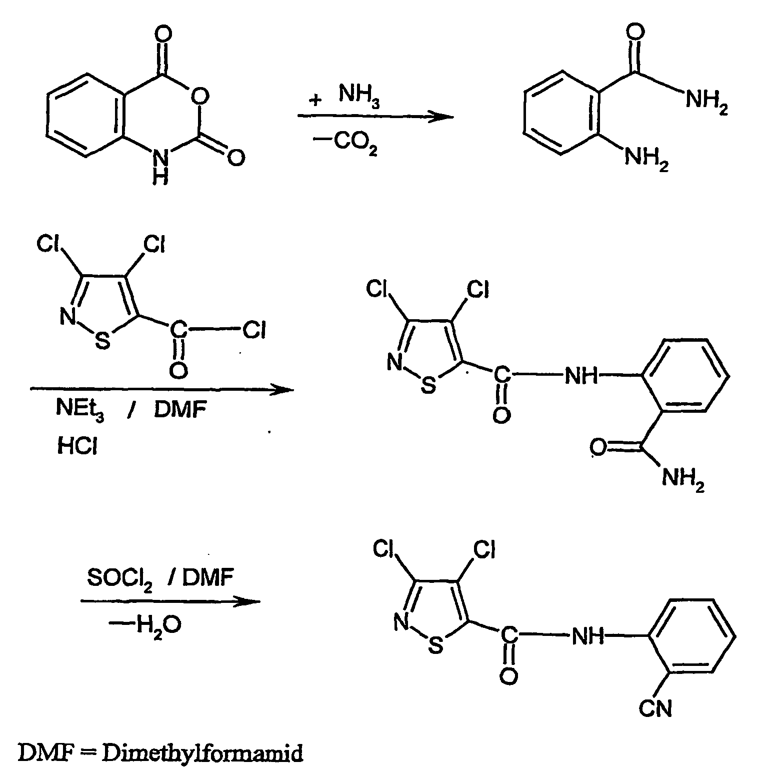

Das bei der Durchführung des erfindungsgemäßen Verfahrens als Ausgangsprodukt benötigte Isatosäureanhydrid der Formel (II) ist bereits bekannt (vgl. DE-A 26 28 055).

Ebenso ist das als Reaktionskomponente in der zweiten Stufe eingesetzte 3,4-Dichlor-isothiazolcarbonsäurechlorid der Formel (IV) schon bekannt (vgl. US-A 5 240 951). Gleiches gilt für das intermediär auftretende Anthranilsäureamid der Formel (III).

Neu hingegen ist das in der dritten Stufe des erfindungsgemäßen Verfahrens als Ausgangssubstanz dienende N-[2-(Aminocarbonyl)-phenyl]-3,4-dichlor-isothiazolcarboxamid der Formel (V).

Bei der Durchführung der ersten Stufe des erfindungsgemäßen Verfahrens dient Ammoniak als Reaktionskomponente. Es wird in trockenem Zustand in das Reaktionsgemisch eingeleitet.

Als Säureakzeptoren kommen bei der Durchführung der zweiten Stufe des erfindungsgemäßen Verfahrens vorzugsweise tertiäre Amine in Frage. Beispielhaft genannt seien Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Bei der Durchführung der dritten Stufe des erfindungsgemäßen Verfahrens fungiert ein Gemisch aus Dimethylformamid und Thionylchlorid, Phosphoroxychlorid, Phosgen oder Chlormethylen-dimethylammoniumchlorid als Dehydratisierungsmittel.

Bei der Durchführung der dritten Stufe des erfindungsgemäßen Verfahrens setzt man Dimethylformamid in einem solchen Überschuss ein, dass es nicht nur als Reaktionskomponente, sondern auch als Verdünnungsmittel dient

Die Reaktionstemperaturen können bei der Durchführung des erfindungsgemäßen Verfahrens innerhalb eines größeren Bereiches variiert werden. Im Allgemeinen arbeitet man bei der Durchführung
- der ersten Stufe bei Temperaturen zwischen 0°C und 1.00°C, vorzugsweise zwischen 0°C und 70°C,
- der zweiten Stufe bei Temperaturen zwischen 0°C und 120°C, vorzugsweise zwischen 0°C und 80°C, und
- der dritten Stufe bei Temperaturen zwischen -20°C und +80°C, vorzugsweise zwischen 0°C und 60°C.

Bei der Durchführung des erfindungsgemäßen Verfahrens arbeitet man sowohl bei der Umsetzung in der ersten Stufe als auch der zweiten und dritten Stufe jeweils im Allgemeinen unter Atmosphärendruck. Es ist jedoch auch möglich, jeweils unter erhöhtem Druck zu arbeiten.

Bei der Durchführung des erfindungsgemäßen Verfahrens setzt man im Allgemeinen in der ersten Stufe auf 1 mol an Isatosäureanhydrid der Formel (II) einen hohen Überschuss an gasförmigem Ammoniak ein, fügt dann in der zweiten Stufe zwischen 0,8 und 1,0 mol an 3,4-Dichlor-isothiazolcarbonsäurechlorid der Formel (IV) sowie eine äquivalente Menge oder auch einen Überschuss an Säureakzeptor hinzu und setzt schließlich in der dritten Stufe mit 1 bis 2 mol an Dehydratisierungsmittel um.

Im Einzelnen verfährt man im Allgemeinen in der Weise, dass man in der ersten Stufe zunächst trockenes Ammoniakgas in eine Lösung von Isatosäureanhydrid der Formel (II) in Dimethylformamid einleitet und dann mit Hilfe eines Inertgasstromes das überschüssige Ammoniak entfernt. Danach tropft man in der zweiten Stufe eine Lösung aus 3,4-Dichlor-isothiazolcarbonsäurechlorid der Formel (IV) in Dimethylformamid in das Reaktionsgemisch und fügt anschließend in der dritten Stufe das jeweilige Säurechlorid hinzu. Die Aufarbeitung erfolgt nach üblichen Methoden. Im Allgemeinen geht man in der Weise vor, dass man das Reaktionsgemisch mit Wasser versetzt und den dabei anfallenden Feststoff absaugt, wäscht und trocknet Eine eventuell noch erforderliche Reinigung kann nach üblichen Methoden vorgenommen werden, also zum Beispiel durch Chromatographie oder Umkristallisation.

Das nach dem erfindungsgemäßen Verfahren erhältliche 3,4-Dichlor-N-(2-cyanophenyl)-5-isothiazolcarboxamid der Formel (I) und dessen Verwendung zur Bekämpfung von unerwünschten Mikroorganismen sind bekannt (vgl. WO 99-24 413).

Das erfindungsgemäße Verfahren wird durch die folgenden Beispiele veranschaulicht.

### Beispiele

### Beispiel 1

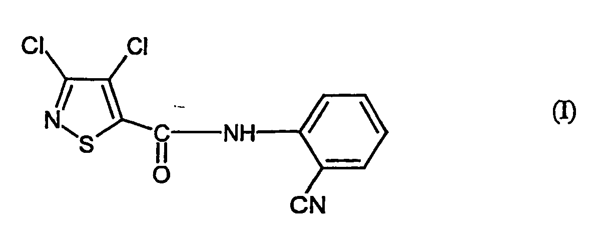

In eine Lösung von 3,59 g (22 mmol) Isatosäureanhydrid in 15 g Dimethylformamid werden bei 60°C unter intensivem Rühren innerhalb von einer Stunde 7 g trockenes Ammoniakgas eingeleitet. Danach entfernt man überschüssiges Ammoniak, indem man 30 Minuten lang Argon durch das auf 60°C erwärmte Reaktionsgemisch leitet. Das Reaktionsgemisch wird dann bei 60°C unter Rühren zunächst mit 2,23 g (22 mmol) Triethylamin und danach tropfenweise mit einer Lösung von 4,33 g (20 mmol) 3,4-Dichlor-isothiazolcarbonsäurechlorid in 10 ml Dimethylformamid versetzt. Nach beendeter Zugabe wird eine Stunde bei 60°C nachgerührt. Anschließend werden bei 60°C unter Rühren 3,33 g (28 mmol) Thionylchlorid zugetropft. Man rührt eine weitere Stunde bei 60°C, kühlt dann auf Raumtemperatur ab, versetzt das Reaktionsgemisch mit 40 ml Wasser und rührt 15 Minuten lang nach. Der anfallende Feststoff wird abgesaugt, zweimal mit je 20 ml Wasser gewaschen und getrocknet. Man erhält auf diese Weise 4,44 g eines Feststoffes, der gemäß ¹H-NMR Spektrum zu 92 % aus 3,4-Dichlor-N-(2-cyano-phenyl)-5-isothiazol-carboxamid besteht. Die Ausbeute errechnet sich danach zu 68,4 % der Theorie, bezogen auf eingesetztes Isatosäureanhydrid. Das entspricht einer Ausbeute von 88 % der Theorie pro Stufe.

### Vergeichsbeispiel A

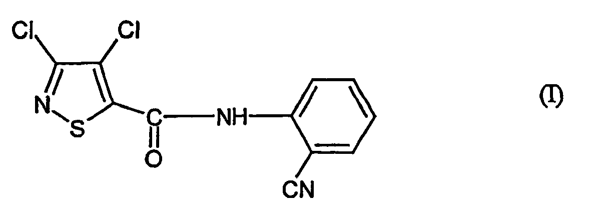

In ein Gemisch aus 20,8 g (0,1725 mol) 2-Cyano-anilin und 250 ml Pyridin werden bei 5 bis 10°C unter Rühren 38,1 g (0,15 mol) 3,4-Dichlor-isothiazol-5-carbonsäurechlorid innerhalb von 10 Minuten eingetropft. Nach beendeter Zugabe versetzt man das Reaktionsgemisch mit 70 ml absolutem Tetrahydrofuran und 30 ml Pyridin, lässt auf Raumtemperatur erwärmen und rührt dann 75 Minuten bei Raumtemperatur. Anschließend wird das Reaktionsgemisch unter vermindertem Druck eingeengt. Der verbleibende Rückstand wird mit 800 ml Wasser und 800 ml Essigsäureethylester verrührt. Der in dem Zweiphasen-Gemisch enthaltene Niederschlag wird abfiltriert, mit Essigsäureethylester gewaschen und getrocknet Man erhält auf diese Weise 31,7 g eines kristallinen Produktes vom Schmelzpunkt 191 bis 193°C.

Aus dem zweiphasigen Filtrat wird die wässrige Phase abgetrennt und zweimal mit je 150 ml Essigsäureethylester extrahiert. Die vereinigten organischen Phasen werden über Natriumsulfat getrocknet und dann unter vermindertem Druck eingeengt. Der verbleibende Rückstand wird mit 100 ml Petrolether und 25 ml Essigsäureethylester verrührt. Der anfallende Feststoff wird abgesaugt, mit Essigsäureethylester nachgewaschen und getrocknet.

Man erhält auf diese Weise insgesamt 40 g (89 % der Theorie) an 3,4-Dichlor-N-(2-cyano-phenyl)-5-isothiazolcarboxamid in Form einer Festsubstanz vom Schmelzpunkt 191 bis 193°C.

## Patentansprüche

1. Verfahren zur Herstellung von 3,4-Dichlor-N-(2-cyano-phenyl)-5-isothiazolcarboxamid der Formel 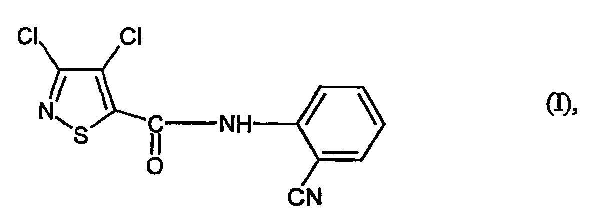 **dadurch gekennzeichnet, dass** man nach einem Eintopi-Verfahren
a) zunächst Isatosäureanhydrid der Formel 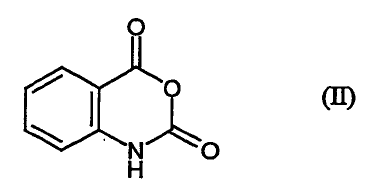 in Gegenwart von Dimethylformamid mit Ammoniakgas umsetzt,
b) dann das dabei entstandene Anthranilsäureamid der Formel 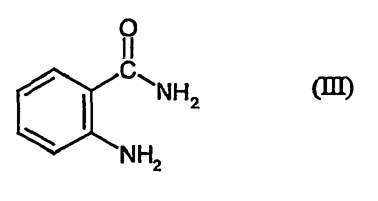 ohne vorherige Isolierung mit 3,4-Dichlor-isothiazolcarbonsäurechlorid der Formel. 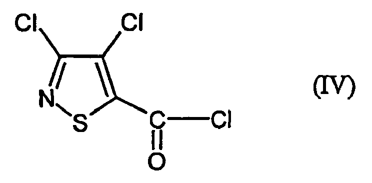 in Gegenwart eines Säureakzeptors und in Gegenwart von Dimethylformamid umsetzt und
c) anschließend das dabei entstandene N-[2-(Aminocarbonyl)-phenyl]-3,4-dichlor-isothiazolcarboxamid der Formel 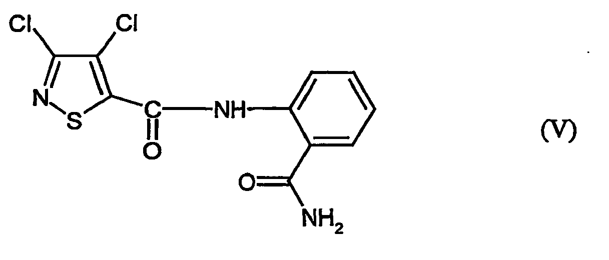 ohne vorherige Isolierung in Gegenwart von Dimethylformamid mit Thionylchlorid, Phosphoroxychlorid, Phosgen oder Chlormethylen-dimethylammoniumchlorid umsetzt

2. Verfahren gemäß Anspruch 1, **dadurch gekennzeichnet, dass** man bei der Durchführung der dritten Stufe Dimethylformamid im Gemisch mit Thionylchlorid als Dehydratisierungsmittel einsetzt.

3. Verfahren gemäß Anspruch 1, **dadurch gekennzeichnet, dass** man bei der Durchführung der ersten Stufe bei Temperaturen zwischen 0°C und 70°C arbeitet

4. Verfahren gemäß Anspruch 1, **dadurch gekennzeichnet, dass** man bei der Durchführung der zweiten Stufe bei Temperaturen zwischen 0°C und 120°C arbeitet.

5. Verfahren gemäß Anspruch 1, **dadurch gekennzeichnet, dass** man bei der Durchführung der dritten Stufe bei Temperaturen zwischen -20°C und +80°C arbeitet.

## Claims

1. Process for preparing 3,4-dichloro-N-(2-cyanophenyl)-5-isothiazolecarboxamide of the formula 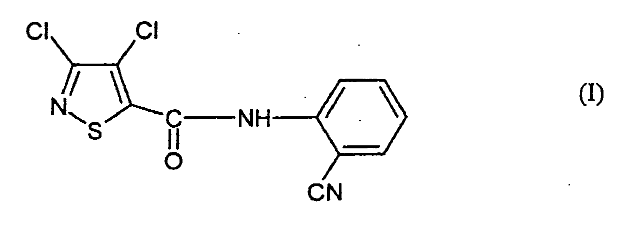 **characterized in that**, in a one-pot process,
a) initially isatoic anhydride of the formula 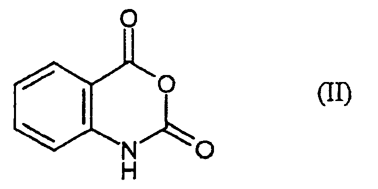 is reacted with ammonium gas in the presence of dimethylformamide,
b) the resulting anthranilamide of the formula 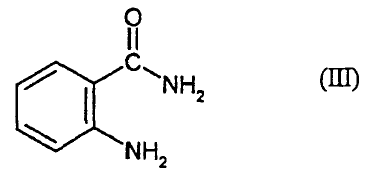 is then, without prior isolation, reacted with 3,4-dichloroisothiazolecarbonyl chloride of the formula 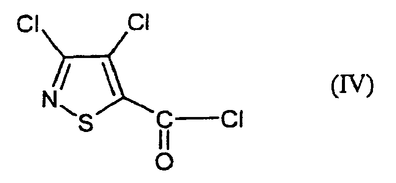 in the presence of an acid acceptor and in the presence of dimethylformamide and,
c) the resulting N-[2-(aminocarbonyl)phenyl]-3,4,-dichloroisothiazolecarboxamide of the formula 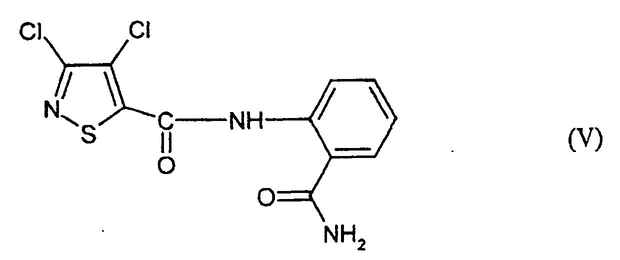 is then, without prior isolation, reacted in the presence of dimethylformamide with thionyl chloride, phosphorus oxychloride, phosgene or chloromethylenedimethylammonium chloride.

2. Process according to Claim 1, **characterized in that** a mixture of dimethylformamide and thionyl chloride as dehydrating agent is used for carrying out the third step.

3. Process according to Claim 1, **characterized in that** the first step is carried out at temperatures between 0°C and 70°C.

4. Process according to Claim 1, **characterized in that** the second step is carried out at temperatures between 0°C and 120°C.

5. Process according to Claim 1, **characterized in that** the third step is carried out at temperatures between -20°C and +80°C.

## Revendications

1. Procédé de production de 3,4-dichloro-N-(2-cyanophényl)-5-isothiazolcarboxamide de formule 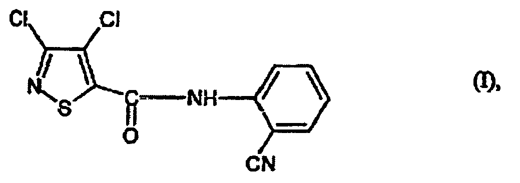 **caractérisé en ce que**, selon un mode opératoire en récipient unique
a) on fait tout d'abord réagir l'anhydride d'acide isatique de formule 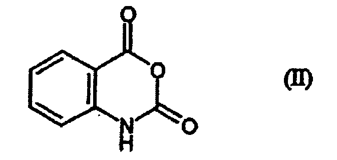 en présence de diméthylformamide, avec l'ammoniac gazeux,
b) puis on fait réagir l'amine d'acide anthranilique ainsi produit, de formule 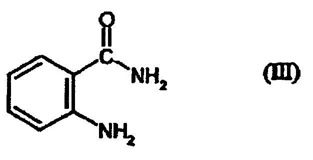 sans isolement préalable, avec le chlorure d'acide 3,4-dichlorisothiazolcarboxylique de formule 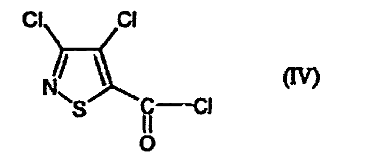 en présence d'un accepteur d'acide et en présence de diméthylformamide, puis
c) on fait réagir le N-[2-(aminocarbonyl)-phényl)]-3,4-dichlorisothiazolcarboxamide ainsi formé, de formule 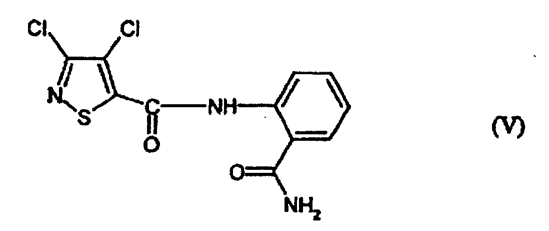 sans isolement préalable, en présence de diméthylformamide, avec le chlorure de thionyle, l'oxychlorure de phosphore, le phosgène ou le chlorure de chlorométhylènediméthylammonium.

2. Procédé suivant la revendication 1, **caractérisé en ce qu'**on utilise le diméthylformamide en mélange avec le chlorure de thionyle comme agent déshydratant dans la conduite de la troisième étape.

3. Procédé suivant la revendication 1, **caractérisé en ce qu'**on opère à des températures comprises entre 0°C et 70°C dans la conduite de la première étape.

4. Procédé suivant la revendication 1, **caractérisé en ce qu'**on opère à des températures comprises entre 0°C et 120°C dans la conduite de la deuxième étape.

5. Procédé suivant la revendication 1, **caractérisé en ce qu'**on opère à des températures comprises entre -20°C et +80°C dans la conduite de la troisième étape.
